# EUROPÄISCHE PATENTSCHRIFT

(11) **EP 0 579 021 B1**
(45) Veröffentlichungstag und Bekanntmachung des Hinweises auf die Patenterteilung: **14.01.1998**
(21) Anmeldenummer: 93110268.5
(22) Anmeldetag: 28.06.1993
(51) Int. Cl.: C07C 17/363

(54) **Verfahren zur Herstellung von Halogenaromaten**
Process for preparing haloaromatics
Procédé de préparation d'aromates halogénés

(30) Priorität: 09.07.1992 DE 4222517
(43) Veröffentlichungstag der Anmeldung: 19.01.1994
(73) Patentinhaber: BAYER AG, 51368 Leverkusen (DE)
(72) Erfinder: Lui, Norbert, Dr., D-51061 Köln (DE); Marhold, Albrecht, Dr., D-51373 Leverkusen (DE)

(56) Entgegenhaltungen:
- EP-A- 0 118 241
- EP-A- 0 427 603
- US-A- 3 283 018

## Beschreibung

Die vorliegende Erfindung betrifft ein Verfahren zur Herstellung von Halogenaromaten durch katalytische Decarboxylierung von aromatischen Halogenameisensäureestern in der Gasphase.

Es ist bekannt, daß man bei der Chlorierung von m-Dialkylbenzolen Isomerengemische erhält, die nur schwer auftrennbar sind und das jeweilige 2,6-Dialkylchlorbenzol nur als Nebenkomponente enthalten (siehe J.Org. Chem. 55, 5260-5269 (1990)).

Man kann 2,6-Dialkylfluorbenzol selektiver herstellen, wenn man in m-Dialkylbenzol in m-Stellung zu den beiden Alkylgruppen zunächst eine tertiäre Butylgruppe einführt, dann nitriert, reduziert, diazotiert, in Gegenwart von Fluoridionen verkocht und dann die tertiäre Butylgruppe wieder abspaltet (siehe J. Chem. Soc. Perkin Trans. I 1987, 1). Nachteilig ist der vielstufige Verfahrensweg.

Eine andere Synthesemöglichkeit für 2,6-Dialkylhalogenbenzole geht von 2,3-Dimethylbutadien aus, das mit Dichlorcarben umgesetzt, umgelagert, mit Triphenylzinnwasserstoff dechloriert und nochmals mit Dichlorcarben umgesetzt wird (siehe Synthesis, Band 6-7, Seiten 647 bis 649). Das hierfür benötigte 2,3-Dimethylbutadien ist schwierig zugänglich und damit teuer. Der Verfahrensweg ist auch hier sehr umständlich und deshalb höchstens von präparativem Interesse.

Aliphatische Fluor- und Chlorameisensäureester lassen sich in Gegenwart von Lewis-Säuren thermisch zu den entsprechenden Fluor- und Chloralkanen umsetzen (siehe DE-OS 2 931 777, US-PS 4 814 524 und DE-PS 857 350).

Aromatische Fluorameisensäureester lassen sich in Gegenwart von Aluminiumoxid oder edelmetallhaltigem Aluminiumoxid thermisch zu Fluoraromaten umsetzen (siehe EP-OS 118 241). Die erzielbaren Ausbeuten sind dabei nicht befriedigend. Außerdem muß beim Einsatz von Chlorameisensäureestern ein großer Überschuß an Fluorwasserstoff eingesetzt werden, um Fluoraromaten zu erhalten (siehe EP-OS 427 603). Im übrigen wird die Herstellung von chlorierten Aromaten nach dieser Methode nicht beschrieben.

Es wurde nun ein Verfahren zur Herstellung von Halogenaromaten der Formel (I) gefunden 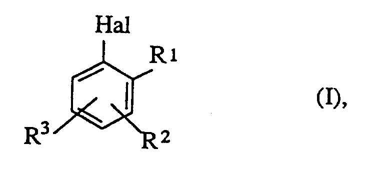 in der
- Hal: für Chlor steht,
- R¹: für Methyl, Ethyl oder i-Propyl steht,
- R²: für Wasserstoff, Methyl, Ethyl oder i-Propyl steht,
- R³: für Wasserstoff, Methyl, Ethyl, i-Propyl, COCH₃, COC₂H₅, Fluor, Chlor, Methoxy, Ethoxy oder i-Propoxy steht oder
- R¹ und R²: gemeinsam eine -CH=CH-CH=CH-Brücke bilden,
das dadurch gekennzeichnet ist, daß man einen Halogenameisensäureester der Formel 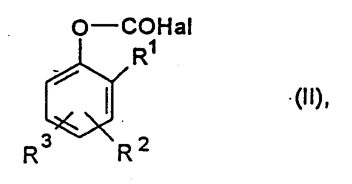 in welcher
- R¹, R², R³ und Hal: die oben angegebenen Bedeutungen haben,
in der Gasphase bei Temperaturen zwischen 200 °C und 500 °C und Drucken zwischen 0,1 und 2 bar über einen durch Chlorwasserstoff aktivierten Al₂O₃-Katalysator leitet.

Die Reste R² und R³ befinden sich vorzugsweise in ortho- oder meta-Positionen zu R¹.

Man wählt die Druck- und Temperaturbedingungen stets so, daß sich die Verbindung der Formel (II) unmittelbar vor der Reaktion am Katalysator in der Gasphase befindet.

Bei den erfindungsgemäß einzusetzenden Katalysatoren handelt es sich um Al₂O₃-Katalysatoren, die mit Chlorwasserstoff aktiviert worden sind.

Der Katalysator liegt vorzugsweise in stückiger Form, z.B. in Teilchen mit einem mittleren Durchmesser von 0,3 bis 5 mm vor.

Die Katalysatoren können beispielsweise ausgehend von stückigem γ-Al₂O₃ hergestellt werden, das man durch Überleiten von gasförmigem Chlorwasserstoff aktiviert. Das Überleiten kann beispielsweise bei Temperaturen zwischen 200 und 500°C erfolgen. Bezogen auf 100 ml Aluminiumoxid kann man beispielsweise von 1 bis 100 g/h Chlorwasserstoff für einen Zeitraum von 0,1 bis 8 Stunden anwenden. Es ist vorteilhaft, das Aluminiumoxid vor dem Überleiten von Chlorwasserstoff zu trocknen, beispielsweise durch von Überleiten von Stickstoff bei erhöhten Temperaturen.

Die Verbindung der Formel (II) kann man alleine oder im Gemisch mit inerten Gasen, z.B. Stickstoff, über den Katalysator leiten. Pro Stunde kann man bezogen auf 100 g Katalysator beispielsweise 5 bis 100 g einer Verbindung der Formel (II) über den Katalysator leiten.

Das nach dem Überleiten der Verbindung der Formel (II) über den Katalysator vorliegende heiße Gasgemisch kann man beispielsweise aufarbeiten, indem man es abkühlt, beispielsweise die bei 50°C oder darunter kondensierbaren Anteile des Gasgemisches kondensiert und aus dem Kondensat die hergestellte Verbindung der Formel (I) z.B. durch Destillation isoliert.

Mit Hilfe des erfindungsgemäßen Verfahrens kann man auf vorteilhafte Weise aromatische Halogenameisensäureester thermisch/katalytisch zu den entsprechenden Halogenaromaten zersetzen. Dabei können Chloraromaten selektiv ohne die Bildung weiterer Isomerer erhalten werden.

Die auf erfindungsgemäße Weise zugänglichen Halogenaromaten der Formel (I) werden als Zwischenprodukte für die Herstellung von Pflanzenschutzmitteln benötigt (siehe DE-A 4 128 132).

### Beispiele

### Beispiel 1

a) Herstellung eines Katalysators
   In einem 35 cm langen Quarzrohr (Durchmesser 25 mm) mit elektrischer Heizwicklung wurden 300 ml γ-Al₂O₃ in einem Stickstoffstrom bei 450°C 48 Stunden lang getrocknet und anschließend 16 Stunden lang trockener Chlorwasserstoff in einer Menge von 60 g/h hindurchgeleitet.
b) Umsetzung von 2,6-Dimethylphenylchlorameisensäureester
   Über einen Vorverdampfer, bestehend aus einem 20 cm langen Quarzrohr (Durchmesser 25 mm), gefüllt mit Quarzstücken und beheizt auf 280°C wurden stündlich 30 g 2,6-Dimethylphenylchlorameisensäureester in das unter a) beschriebene Quarzrohr geleitet, das den wie unter a) beschriebenen aktivierten Katalysator enthielt. Die Reaktionstemperatur betrug 230°C. Gleichzeitig wurden 24 l/h Stickstoff durch das Reaktionsgefäß geleitet. Die das Reaktionsgefäß verlassenden Gase wurden in einem Kühler bei 20°C kondensiert 2-Chlor-1,3-dimethylbenzol bildete sich während eines 16-stündigen kontinuierlichen Betriebs mit einer Selektivität von über 95 % und in einer Ausbeute von 85 %.

### Beispiel 2

Es wurde verfahren wie in Beispiel 1, jedoch wurden stündlich 20 g 2,3-Dimethylphenylchlorameisensäureester eingesetzt. Es wurde 2,3-Dimethylchlorbenzol mit einer Selektivität von über 90 % gebildet. Das im Kühler aufgefangene Kondensat wurde mit 5 gew.-%iger wäßriger Natronlauge gewaschen und im Vakuum destilliert. So wurde 2,3-Dimethylchlorbenzol mit einem Siedepunkt von 187°C bei 1030 mbar in einer Ausbeute von 54 % erhalten.

### Beispiel 3

Es wurde verfahren wie in Beispiel 1, jedoch wurden 20 g 2,4,6-Trimethylphenylchlorameisensäureester eingeleitet. Nach Kondensation des Reaktionsgases, Waschen mit Wasser und Destillation im Vakuum wurde in einer Ausbeute von 74 % und mit einem Siedepunkt von 120°C bei 72 mbar 2,4,6-Trimethylchlorbenzol erhalten.

### Beispiel 4

Es wurde verfahren wie in Beispiel 1, jedoch wurden 20 g 2,4-Dimethylphenylchlorameisensäureester eingeleitet. Die Reaktionsgase wurden in einem Kühler kondensiert, mit Wasser gewaschen und im Vakuum destilliert. Mit einer Ausbeute von 31 % wurde 2,4-Dimethylchlorbenzol erhalten, das einen Siedepunkt von 186°C bei Normaldruck aufwies.

### Beispiel 5

Es wurde verfahren wie in Beispiel 1, jedoch wurden 20 g 1-Naphthalinchlorameisensäureester eingeleitet. Dieses wurde zu 40 % umgesetzt und im Reaktionsgas 1-Chlomaphthalin in einer Ausbeute von 34 % der Theorie analysiert.

### Vergleichsbeispiel 1

150 g γ-Al₂O₃ wurden in einem Quarzrohr (35 cm lang, Durchmesser 25 mm) in einem Stickstoffstrom 36 h lang bei 200°C getrocknet Danach wurden bei 200°C 20 g/h 2,6-Dimethylphenylchlorameisensäureester durch das Quarzrohr geleitet, die Reaktionsgase bei -20°C kondensiert und gaschromatographisch untersucht. In den beiden ersten Betriebsstunden betrug der Umsatz 100 %, danach fiel er auf 95 % ab, gleichzeitig bildete sich Dimethylphenol zu einem Anteil von 20 % (bezogen auf Chlorameisensäureester).

### Vergleichsbeispiel 2

Es wurde verfahren wie in Beispiel 1, jedoch ein γ-Al₂O₃ eingesetzt, das 30 g/l Eisen(III)-chlorid enthielt. Die Ergebnisse wichen nur wenig von denen des Vergleichsbeispiels 1 ab.

## Patentansprüche

1. Verfahren zur Herstellung von Halogenaromaten der Formel 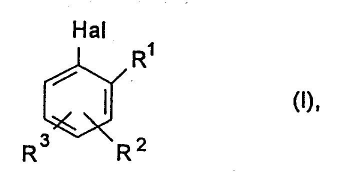 in der
Hal für Chlor steht,
R¹ für Methyl, Ethyl oder i-Propyl steht,
R² für Wasserstoff, Methyl, Ethyl oder i-Propyl steht,
R³ für Wasserstoff, Methyl, Ethyl, i-Propyl, COCH₃, COC₂H₅, Fluor, Chlor, Methoxy, Ethoxy oder i-Propoxy steht oder
R¹ und R² gemeinsam eine -CH=CH-CH=CH-Brücke bilden,
dadurch gekennzeichnet, daß man einen Halogenameisensäureester der Formel 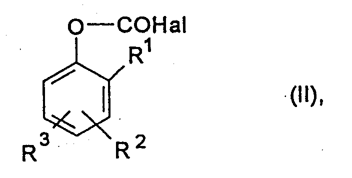 in welcher
R¹, R², R³ und Hal die oben angegebenen Bedeutungen haben,
in der Gasphase bei Temperaturen zwischen 200 °C und 500 °C und Drucken zwischen 0,1 und 2 bar über einen durch Chlorwasserstoff aktivierten Al₂O₃-Katalysator leitet.

2. Verfahren nach Anspruch 1, dadurch gekennzeichnet, daß sich bei den Formeln (I) und (II) R² und R³ in ortho- oder meta-Positionen zu R¹ befinden.

3. Verfahren nach Anspüchen 1 bis 2, dadurch gekennzeichnet, daß der Katalysator ausgehend von stückigem γ-Al₂O₃ hergestellt worden ist, das man durch Überleiten von gasförmigen Chlorwasserstoff aktiviert hat.

4. Verfahren anch Ansprüchen 1 bis 3, dadurch gekennzeichnet, daß man die Verbindung der Formel (II) alleine oder im Gemisch mit inerten Gasen über den Katalysator leitet.

5. Verfahren nach Ansprüchen 1 bis 4, dadurch gekennzeichnet, daß man, bezogen auf 100 g Katalysator, stündlich 5 bis 100 g einer Verbindung der Formel (II) über den Katalysator leitet.

6. Verfahren nach Ansprüchen 1 bis 5, dadurch gekennzeichnet, daß man das nach dem Überleiten über den Katalysator vorliegende heiße Gasgemisch aufarbeitet, indem man es abkühlt, die kondensierbaren Anteile des Gasgemisches kondensiert und aus dem Kondensat die hergestellte Verbindung der Formel (I) isoliert.

## Claims

1. Process for the preparation of halogenated aromatic compounds of the formula 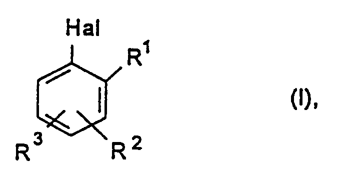 in which
Hal represents chlorine,
R¹ represents methyl, ethyl or i-propyl,
R² represents hydrogen, methyl, ethyl or i-propyl,
R³ represents hydrogen, methyl, ethyl, i-propyl, COCH₃, COC₂H₅, fluorine, chlorine, methoxy, ethoxy or i-propoxy or
R¹ and R² together form a -CH=CH-CH=CH- bridge,
characterised in that a halogenoformic ester of the formula 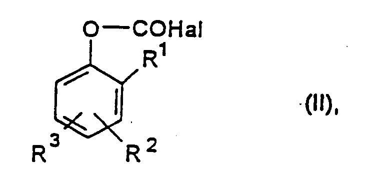 in which
R¹, R², R³ and Hal have the meanings given above,
is passed in the gas phase over an Al₂O₃ catalyst activated by hydrogen chloride at temperatures between 200°C and 500°C and pressures between 0.1 and 2 bar.

2. Process according to Claim 1, characterised in that, in the formulae (I) and (II), R² and R³ are in ortho- or meta- positions to R¹.

3. Process according to Claims 1 to 2, characterised in that the catalyst has been prepared starting from particulate γ-Al₂O₃ which was activated by passing over gaseous hydrogen chloride.

4. Process according to Claims 1 to 3, characterised in that the compound of the formula (II) is passed over the catalyst alone or in a mixture with inert gases.

5. Process according to Claims 1 to 4, characterised in that, based on 100 g of catalyst, 5 to 100 g of a compound of the formula (II) are passed over the catalyst each hour.

6. Process according to Claims 1 to 5, characterised in that the hot gas mixture present after gases have been passed over the catalyst is worked up by cooling it, condensing the condensable portions of the gas mixture and isolating from the condensate the compound prepared of the formula (I).

## Revendications

1. Procédé pour la préparation de composés aromatiques halogénés répondant à la formule 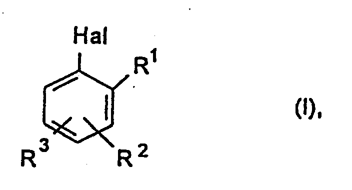 dans laquelle
Hal représente un atome de chlore,
R¹ représente un groupe méthyle, un groupe éthyle ou un groupe i-propyle,
R² représente un atome d'hydrogène, un groupe méthyle, un groupe éthyle ou un groupe i-propyle,
R³ représente un atome d'hydrogène, un groupe méthyle, un groupe éthyle, un groupe i-propyle, COCH₃, COC₂H₅, un atome de fluor, un atome de chlore, un groupe méthoxy, un groupe éthoxy ou un groupe i-propoxy, ou bien
R¹ et R² forment ensemble un pont -CH=CH-CH=CH-,
caractérisé en ce qu'on guide un halogénoformiate répondant à la formule 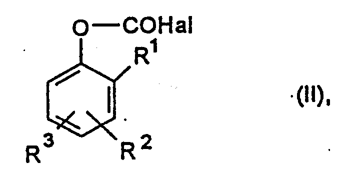 dans laquelle
R¹, R², R³ et Hal ont les significations indiquées ci-dessus,
en phase gazeuse, à des températures entre 200°C et 500°C et sous des pressions entre 0,1 et 2 bar, par-dessus un catalyseur de Al₂O₃ activé par de l'acide chlorhydrique.

2. Procédé selon la revendication 1, caractérisé en ce que R² et R³ dans les formules (I) et (II) se trouvent dans des positions ortho ou méta par rapport à R¹.

3. Procédé selon les revendications 1 à 2, caractérisé en ce que le catalyseur a été préparé à partir de γ-Al₂O₃ en morceaux, que l'on a activé en faisant passer par-dessus du gaz chlorhydrique.

4. Procédé selon les revendications 1 à 3, caractérisé en ce qu'on guide le composé de formule (II), seul ou en mélange avec des gaz inertes, par-dessus le catalyseur.

5. Procédé selon les revendications 1 à 4, caractérisé en ce qu'on guide, rapportés à 100 g de catalyseur, par heure, de 5 à 100 g d'un composé de formule (II) par-dessus le catalyseur.

6. Procédé selon les revendications 1 à 5, caractérisé en ce qu'on traite le mélange gazeux chaud présent après son guidage par-dessus le catalyseur, en le refroidissant, en condensant les fractions condensables du mélange gazeux et en isolant du condensat le composé préparé de formule (I).
